(19) Europäisches Patentamt
European Patent Office
Office européen des brevets

(11) Veröffentlichungsnummer: **0 035 473**
**B1**

(12) # EUROPÄISCHE PATENTSCHRIFT

(45) Veröffentlichungstag der Patentschrift: **22.02.84**

(51) Int. Cl.³: **C 07 C 149/20,** C 08 K 5/37

(21) Anmeldenummer: **81810060.4**

(22) Anmeldetag: **25.02.81**

(54) **Mercaptophenole und ihre Verwendung als Stabilisatoren.**

(30) Priorität: **03.03.80 CH 1665/80**

(43) Veröffentlichungstag der Anmeldung: **09.09.81 Patentblatt 81/36**

(45) Bekanntmachung des Hinweises auf die Patenterteilung: **22.02.84 Patentblatt 84/8**

(84) Benannte Vertragsstaaten: **DE FR GB IT**

(56) Entgegenhaltungen:
**DE - A - 2 659 406**
**GB - A - 2 013 668**
**US - A - 4 021 468**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Rosenberger, Siegfried, Im Gehracker 11, CH-4125 Riehen (CH)**
Erfinder: **Evans, Samuel, Dr., Schützenrain 3, CH-4125 Riehen (CH)**
Erfinder: **Gilg, Bernard, Dr., Route de Bâle, F-68300 Saint-Louis (FR)**

**EP 0 035 473 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Mercaptophenole und ihre Verwendung als Stabilisatoren

Die Erfindung betrifft neue Mercaptophenole, welche als Stabilisatoren für organisches Material Verwendung finden.

Es sind bereits Phenolgruppen und Mercaptogruppen enthaltende Stabilisatoren für organisches Material, insbesondere für Polymere, bekannt, welche beispielsweise nach Einmischen in das zu stabilisierende Material ihre Wirkung entfalten. Im Falle der Stabilisierung von Polymeren, insbesondere gegen Oxidation, kann die Stabilisierung sehr vorteilhaft in der Weise erfolgen, daß man den jeweiligen Stabilisator in Gegenwart eines Radikalbildners auf das Polymer aufpfropft. Derartige Stabilisator-Fixierungen auf Polymeren sind ausführlich in der DE-OS 2 509 654 beschrieben. Als weitere einschlägige Publikationen sind eine Notiz in Chemical Age 17/24, August 1979, Seite 5, und eine Abhandlung in British Plastic and Rubber, April 1977, Seiten 25–26, anzuführen. In der letztgenannten Publikation werden auf Seite 26 noch weitere Publikationen von Gerald Scott in verschiedenen Zeitschriften angegeben.

Außerdem ist auch noch auf eine japanische Publikation »Network bound antioxidants in diene rubber« in Nippon Gomu Kyokaishi 1977, 50(8), 548–552, (Chem. A. 87, (1977) 44 (168997a) und die DE-OS 2 659 406 hinzuweisen.

Die Bindung von Stabilisatoren an die Polymeren, welche über Schwefelbrücken erfolgt, bringt folgende Vorteile mit sich:

a) Die Stabilisatoren gehen weder durch Verflüchtigung noch durch Extraktionsvorgänge verloren.
b) Niedere oder fehlende Migration bringt Vorteile toxikologischer Art mit sich.

Die erfindungsgemäßen Mercaptophenole zeigen nach ihrer Aufpfropfung auf vorgegebene Polymere bessere Wirksamkeiten, verglichen mit den vorbekannten, ähnlichen Verbindungen.

Gegenstand der Erfindung sind Verbindungen der Formel I

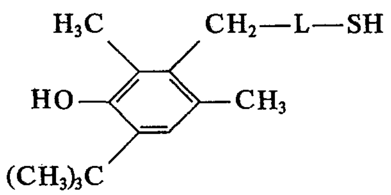

(I)

worin L einen der 2wertigen Reste $-SCH_2CO \cdot OCH_2CH_2-$

$$-SCH_2CO \cdot O(CH_2CH_2S)_yCH_2CH_2- \qquad -O \cdot CO \cdot C_pH_{2p}-$$

$$-SCH_2CH_2O \cdot CO \cdot C_pH_{2p}- \qquad -OC_tH_{2t}NH \cdot CO \cdot C_pH_{2p}-$$

$$-NH-C_tH_{2t}NH \cdot CO \cdot C_pH_{2p}- \qquad -S \cdot C_pH_{2p}- \qquad -NH \cdot CO \cdot C_pH_{2p}-$$

$$-NH-C_pH_{2p}- \qquad -O \cdot C_pH_{2p}- \qquad -OC_tH_{2t}OCO \cdot C_pH_{2p}-$$

$$-NH \cdot C_tH_{2t}O \cdot CO \cdot C_pH_{2p}-$$

y eine Zahl von 1 bis 3 und p und t eine Zahl von 1 bis 18 bedeuten.

Besonders bevorzugte Verbindungen der Formel I sind die der folgenden Struktur

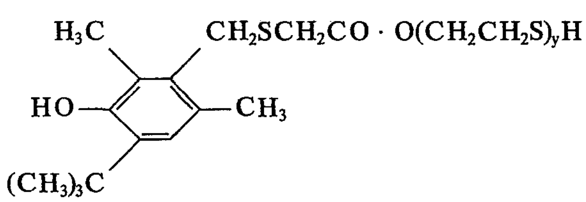

worin y 1 oder 2 bedeutet.

Die Herstellung der erfindungsgemäßen Verbindungen erfolgt nach an sich bekannten Verfahren. Für Verbindungen der Formel I gilt etwa folgende Formelgleichung:

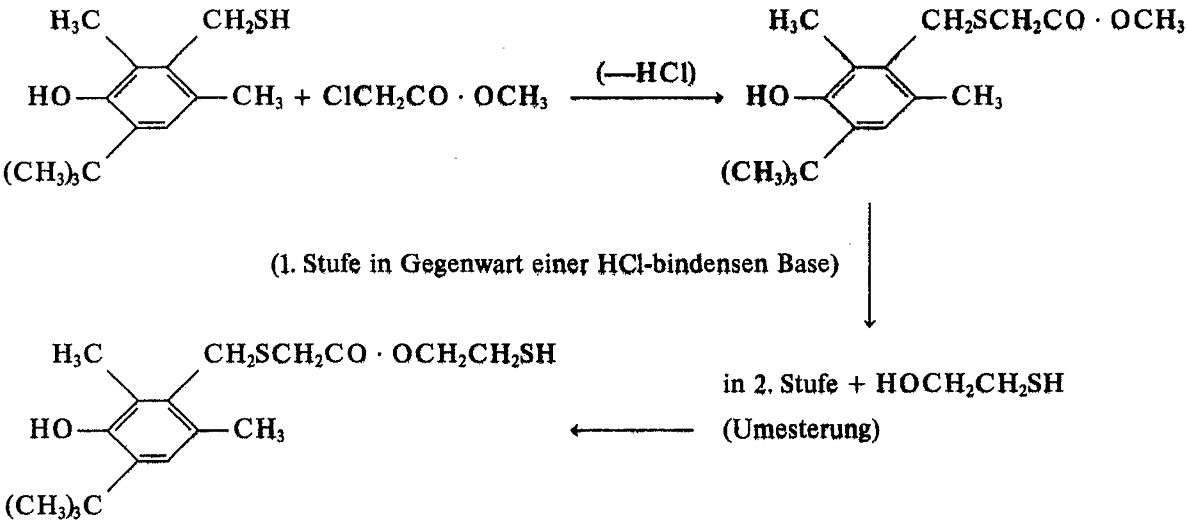


Als Ausgangsstoffe für die Herstellung der erfindungsgemäßen Verbindungen werden bekannte Substanzen verwendet, deren Herstellung dem Fachmann ebenfalls bekannt ist.

Weiterer Gegenstand der Erfindung ist die Verwendung der Verbindungen der Formel I als Stabilisatoren für organisches Material, insbesondere zur Herstellung von stabilisierten Polymeren durch Aufpfropfen der Verbindungen auf die Polymeren.

Das Aufpfropfen der erfindungsgemäßen Mercaptophenole auf die vorgegebenen Polymeren erfolgt in der Weise, daß die Polymeren mit den Mercaptophenolen in Gegenwart eines Radikalbildners umgesetzt werden. Die Umsetzung kann in wässeriger Emulsion oder Suspension, in Lösung oder in der Masse erfolgen. Vorteilhaft werden für die Reaktion solche Bedingungen angewandt, die denjenigen der Herstellung des Polymeren ähnlich sind. Eine besondere Bedeutung kommt hier der Pfropfung in wässerige Emulsion zu.

Als Radikalbildner kommen organische Peroxide, Hydroperoxide oder Persulfate und deren Kombinationen mit Aminen oder $Fe^{2\pm}$-Salzen, vorzugsweise Azoverbindungen in Frage. Das Gewichtsverhältnis von Stabilisator zu Radikalbildner ist nicht kritisch und beträgt 100 : 1 bis 0,25 : 1.

Die Umsetzung der Stabilisatoren kann mit all den Polymeren erfolgen, welche in der DE-OS 2 509 654 aufgezählt worden sind. So folgende Polymere: Polyolefine, Polystyrol, Polyvinylchlorid, Polyamide, Polyester, natürliche oder synthetische Kautschukarten, Äthylen-Propylen-Kautschuk, Styrol-Butadien, Acrylnitril-Butadien-Styrol, Polybutadien, Polyisopren, Methacrylat-Butadien-Styrol- und Methylmethacrylat-Butadien-Styrol-Copolymere, besonders in Form von Latices. Natürlich können auch Gemische von Polymeren angewandt werden. Die Polymere besitzen normalerweise ein hohes Molekulargewicht, z. B., daß das Polymer film- oder faserbildend ist; aber niedermolekulare Polymere, z. B. Polymere, die noch flüssig sind, können ebenfalls angewandt werden, wenn die Addukte als Zusätze für andere Polymere verwendet werden sollen. Als Latices kommen auch folgende Polymere in Frage: NR, BR, NBR, SBR, CR, MBS, ABS.

Bei der erfindungsgemäßen Verwendung unter Aufpropfung wird das Antioxidans in einer Menge von 0,001 bis 5%, vorzugsweise 0,1 bis 1%, bezogen auf das Gewicht des Polymers, zugegeben, wenn die Eigenschaften des Polymers modifiziert werden sollen. Wenn andererseits das Antioxidans zugegeben wird, um ein Addukt herzustellen, das angewandt werden kann, um die Eigenschaften eines anderen Polymers zu modifizieren, können größere Mengen angewandt werden. Im vorliegenden Falle, wo das Antioxidans ein Thiol ist, ist es möglich, so große Mengen wie 300 bis 500 Gew.-%, vorzugsweise 50 Gew.-%, zu verwenden. Die Reaktionstemperaturen liegen zwischen 0 und 200° C, vorzugsweise 50 bis 130° C, dagegen bei Latexsystemen zwischen 40 und 60° C. In einem zweiten Schritt werden diese hochprozentigen Pfropfpolymerisate mit dem Ausgangspolymeren oder einem anderen Polymeren verdünnt. Die Verdünnung kann durch Mischen von Latices oder Lösungen, oder auch durch Eincompondieren des hochprozentigen Pfropfpolymerisates erfolgen.

Mit Hilfe der erfindungsgemäßen Verbindungen können durch den beschriebenen Pfropfprozeß die folgenden Polymeren stabilisiert werden:

Polypropylen, Polyäthylen, Polyisopren, auch als Naturkautschuk, Polybutadien, Terpolymere von Äthylen, Propylen und einem Dien Äthylen-Propylen-Kautschuk, Styrol-Butadien, Styrol-Butadien-Styrol-Blockcopolymerisate, Styrol-Butadien-Pfropfcopolymere, Acrylnitril-Butadien-Styrol-Pfropfcopolymere, Polychloropren, Acrylnitril-Butadien-Copolymere, Methacrylat-Butadien-Styrol-Pfropfpolymere.

Die für den Pfropfprozeß verwendeten Ausgangspolymeren können weitere Additive enthalten, wie z. B.

Weitere alkylierte Phenole als Antoxidantien, Lichtschutzmittel, wie 2-(2'-Hydroxyphenyl)-benztriazole, 2-Hydroxybenzophenone, Nickelverbindungen, sterisch gehinderte Ancine, Phosphite, Thioäther, Füllstoffe, Weichmacher, Gleitmittel, Flammschutzmittel, Antistatika.

Weitere Gegenstände der Erfindung sind somit auch noch:

a) Die erfindungsgemäße Verwendung, dadurch gekennzeichnet, daß man die Verbindungen der Formel I in Gegenwart eines Radikalbildners auf ein Polymeres aufpfropft.
b) Durch Verbindungen der Formel I stabilisierte organische Polymere.
c) Die so stabilisierten Polymeren, dadurch gekennzeichnet, daß sie als Stabilisatoren Verbindungen der Formel I enthalten.
d) Stabilisierte Polymere, dadurch gekennzeichnet, daß sie die Stabilisatoren aufgepfropft enthalten.

## Herstellungsbeispiele

### Beispiel 1

S-(3,5-Dimethyl-4-t-butyl-3-hydroxy-benzyl)-thioglykolsäure-5-mercapto-3-thia-n-pentylester

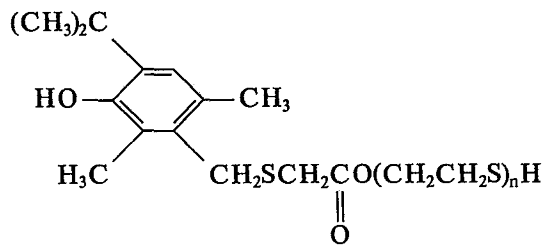

$n = 2$

### A. Vorstufe Methylester

22,7 g 2-t-Butyl-4,6-dimethyl-5-chlormethylphenol (hergestellt nach Vorschrift in »Makromolek. Chemie« Bd. 9, S. 21 – 22 [1952]) werden in 100 ml Dimethylacetamid (DMA) gelöst und diese Lösung unter Rühren in ein Gemisch aus 50 ml DMA, 14 ml Triäthylamin und 12,0 g Thioglykolsäuremethylester eingetropft, wobei die Temperatur des Gemisches auf ca. 35°C ansteigt. Der Ansatz wird 6 Stunden auf 75°C erhitzt. Chromatographisch ist danach praktisch kein Edukt mehr nachweisbar und ein einheitlicher durch Elementaranalysen belegter S-(3,5-Dimethyl-4-t-butyl-3-hydroxybenzyl)-thioglykolsäure-methylester entstanden. Das Lösungsmittel wird im Vakuum abdestilliert und Triäthylaminhydrochlorid durch Wasserbehandlung des obigen Produktes entfernt.

### B. Umesterung mit Mercaptoäthanol zum erfindungsgemäßen Endprodukt

29,6 g Methylester-Vorstufe A werden mit 23,4 g 2-Mercaptoäthanol unter Zusatz von 0,5 ml Tetrabutyl-ortho-titanat 14 h auf 140°C erhitzt ($N_2$, Rühren). Danach wird das überschüssige 2-Mercaptoäthanol im Hochvakuum abdestilliert.

Der viskose Rückstand enthält lt. Methoxyl-Bestimmung nur noch wenig Methylester und besteht nach dem NMR-Spektrum aus einem Gemisch von Mercaptoestern der oben angegebenen Struktur mit $n = 1 - 4$. Aus diesem Gemisch wird die Verbindung dieses Beispiels mit $n = 2$ chromatographisch als viskoses Öl abgetrennt und durch Elementaranalyse und SH-Titration definiert.

Beispiel 2

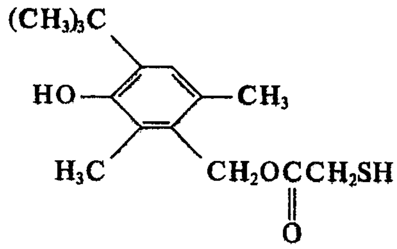

Thioglykolsäure-3-hydroxy-4-t-butyl-2,6-dimethyl-benzylester

22,6 g 2-t-Butyl-4,6-dimethyl-5-chlormethylphenol (hergestellt nach Vorschrift in »Makromolek. Chemie« Bd. 9, S. 21 – 22 [1952]) und 11,4 g Natriumthioglykolat werden in 200 ml Dimethylacetamid 8 Stunden bei 65° C unter Rühren und Stickstoff umgesetzt. Dünnschichtchromatographisch ist danach praktisch kein Edukt mehr nachweisbar. Das Lösungsmittel wird im Vakuum entfernt und das Rohprodukt in Toluol aufgenommen und durch Wasserwäsche vom NaCl befreit. Das Produkt wird nach säulenchromatogr. Reinigung als zähes Harz erhalten und ist durch SH-Gehalt und Elementar-Analyse definiert.

Verwendungsbeispiele

Beispiel I

100 g Polybutadien-Latex mit 30% Feststoffgehalt werden bei 55° C unter Stickstoff gerührt. Bei dieser Temperatur werden in der angegebenen Reihenfolge die folgenden Substanzen zugesetzt:

a) 1 g Natriumstearat in 100 ml $H_2O$,
b) 0,3 g einer Verbindung A, gemäß Herstellungsbeispiel 1, der Formel

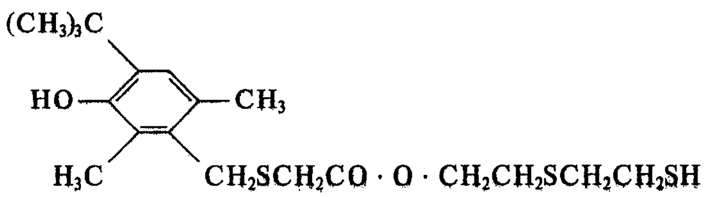

(A)

c) 0,01 g Azo-bis-isobutyronitril.

Das Gemisch wird während 10 Stunden gerührt. Anschließend wird die Emulsion durch Zugabe von 100 ml 2,5%iger Schwefelsäure bei 55° C koaguliert und der ausgefallene Kautschuk gründlich mit Wasser gewaschen. Nach der Filtration wird im Vakuum bei 40° C während 3 Stunden vorgetrocknet. Zur Entfernung des Wassers wird der Kautschuk dreimal durch ein kaltes Walzwerk gelassen. Dann wird bei Raumtemperatur im Vakuum während 12 Stunden getrocknet.

Der trockene Kautschuk wird in einer Heizpresse bei 60° C zu 2 mm dicken Platten verpreßt (Preßzeit 20 Minuten).

Die Prüfung auf Wirksamkeit des zugesetzten Additives wird durch Hitzealterung vorgenommen. Die Hitzealterung besteht im Eintauschen der Prüflinge in Silikonöl bei 160° C während 20 Minuten. Als Kriterium dient der Gelgehalt am Ende der Alterung. Dieser wird folgendermaßen bestimmt:

1 g Polybutadien wird über Nacht bei Raumtemperatur in 100 ml Toluol gelöst. Die Lösung wird durch Glaswolle filtriert und die filtrierte Lösung zur Trockne eingedampft. Der Gelgehalt ergibt sich aus:

$$Gel = \frac{E - W}{E} \times 100(\%)$$

E = Einwaage (üblich 1 g)
W = Gewicht des Eindampfrückstandes

Im vorliegenden Fall zeigen die Versuchsergebnisse der Hitzealterung, daß Gelentwicklung durch das zugesetzte Additiv A wirksam unterdrückt wird.

In einem 2. Versuch wird die günstige Wirkung des Stabilisators ebenfalls durch Alterung nachgewiesen. Die Alterung erfolgt hier aber nach vorheriger 24stündiger Extraktion der Probe mit

Äthanol bei Raumtemperatur.

Die Versuchsergebnisse sind in den Tabellen 1 und 2 veranschaulicht. Sie zeigen auch, daß es sich um sehr extraktionsstabile Stabilisatorsysteme handelt.

Tabelle 1

Alterung ohne vorherige Extraktion mit Äthanol

| | Gelgehalt (%) |
|---|---|
| Ohne Stabilisator | 30 |
| Stabilisator A | 6 |

Tabelle 2

Alterung nach vorheriger Extraktion mit Äthanol

| | Gelgehalt (%) |
|---|---|
| Ohne Stabilisator | 50 |
| Stabilisator A | 5 |

**Patentansprüche**

1. Verbindungen der Formel I

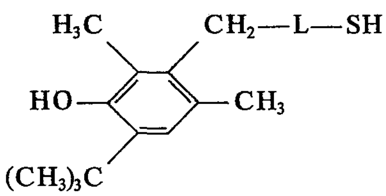

(I)

worin L einen der 2wertigen Reste $-SCH_2CO \cdot OCH_2CH_2-$

$-SCH_2CO \cdot O(CH_2CH_2S)_yCH_2CH_2-$ $\quad -O \cdot CO \cdot C_pH_{2p}-$

$-SCH_2CH_2O \cdot CO \cdot C_pH_{2p}-$ $\quad -OC_tH_{2t}NH \cdot CO \cdot C_pH_{2p}-$

$-NH-C_tH_{2t}NH \cdot CO \cdot C_pH_{2p}-$ $\quad -S \cdot C_pH_{2p}-$ $\quad -NH \cdot CO \cdot C_pH_{2p}-$

$-NH-C_pH_{2p}-$ $\quad -O \cdot C_pH_{2p}-$ $\quad -OC_tH_{2t}OCO \cdot C_pH_{2p}-$

$-NH \cdot C_tH_{2t}O \cdot CO \cdot C_pH_{2p}-$

y eine Zahl von 1 bis 3 und p und t eine Zahl von 1 bis 18 bedeuten.

2. Verbindungen nach Anspruch 1 der Formel I mit folgender Struktur

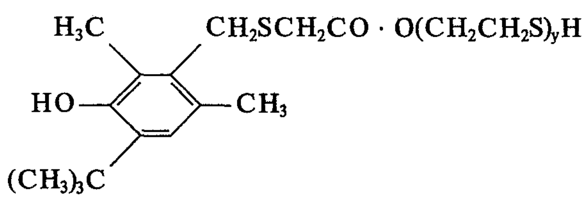

worin y 1 oder 2 bedeutet.

3. Verbindungen nach Anspruch 1 der Formel

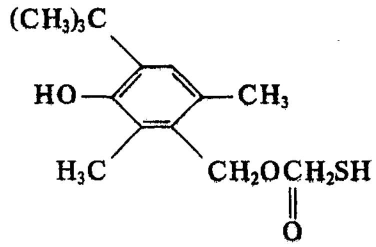

4. Verwendung der Verbindungen nach Anspruch 1 der Formel I als Stabilisatoren für organisches Material.

5. Verwendung nach Anspruch 4, dadurch gekennzeichnet, daß man die Verbindung der Formel I in Gegenwart eines Radikalbildners auf ein Polymer aufpfropft.

6. Durch Verbindungen nach Anspruch 1 stabilisierte organische Polymere.

7. Stabilisierte Polymere nach Anspruch 6, dadurch gekennzeichnet, daß sie die Stabilisatoren aufgepfropft enthalten.

## Claims

1. A compound of the formula I

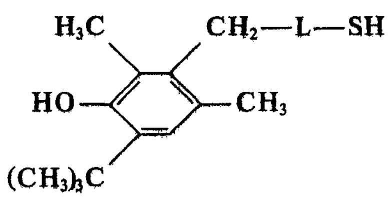

(I)

wherein L is one of the divalent radicals $-SCH_2CO \cdot OCH_2CH_2-$

$-SCH_2CO \cdot O(CH_2CH_2S)_yCH_2CH_2-$ $\quad -O \cdot CO \cdot C_pH_{2p}-$

$-SCH_2CH_2O \cdot CO \cdot C_pH_{2p}-$ $\quad -OC_tH_{2t}NH \cdot CO \cdot C_pH_{2p}-$

$-NH-C_tH_{2t}NH \cdot CO \cdot C_pH_{2p}-$ $\quad -S \cdot C_pH_{2p}-$ $\quad -NH \cdot CO \cdot C_pH_{2p}-$

$-NH-C_pH_{2p}-$ $\quad -O \cdot C_pH_{2p}-$ $\quad -OC_tH_{2t}OCO \cdot C_pH_{2p}-$

$-NH \cdot C_tH_{2t}O \cdot CO \cdot C_pH_{2p}-$

y is a value from 1 to 3 and p and t are a value from 1 to 18.

2. A compound according to claim 1 of the formula I having the structure

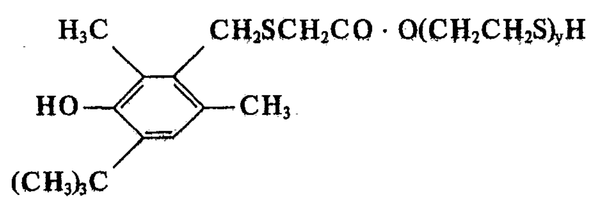

wherein y is 1 or 2.

3. A compound according to claim 1 of the formula

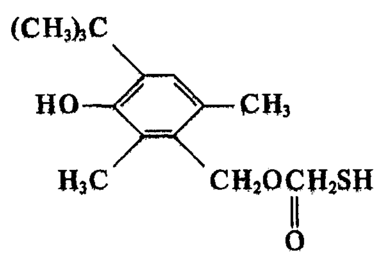

4. A method of stabilising organic material which comprises the use of a compound according to claim 1.

5. A method according to claim 4, which comprises grafting a compound of the formula I, in the presence of a radical initiator, onto a polymer.

6. An organic polymer stabilised by a compound according to claim 1.

7. A stabilised polymer according to claim 6 onto which the stabiliser has been grafted.

**Revendications**

1. Composés répondant à la formule I

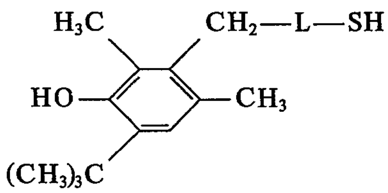

(I)

dans laquelle L représente l'un des radicaux bivalents suivants: $-SCH_2CO \cdot OCH_2CH_2-$

$-SCH_2CO \cdot O(CH_2CH_2S)_yCH_2CH_2-$ $\quad -O \cdot CO \cdot C_pH_{2p}-$

$-SCH_2CH_2O \cdot CO \cdot C_pH_{2p}-$ $\quad -OC_tH_{2t}NH \cdot CO \cdot C_pH_{2p}-$

$-NH-C_tH_{2t}NH \cdot CO \cdot C_pH_{2p}-$ $\quad -S \cdot C_pH_{2p}$ $\quad -NH \cdot CO \cdot C_pH_{2p}-$

$-NH-C_pH_{2p}-$ $\quad -O \cdot C_pH_{2p}-$ $\quad -OC_tH_{2t}OCO \cdot C_pH_{2p}-$ et

$-NH \cdot C_tH_{2t}O \cdot CO \cdot C_pH_{2p}-$

y désigne un nombre de 1 à 3 et p et t représentent chacun un nombre de 1 à 18.

2. Composés de formule I selon la revendication 1 qui répondent à la formule suivante:

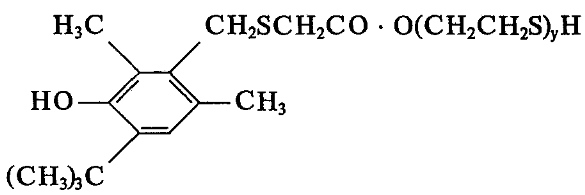

dans laquelle y est égal à 1 ou à 2.

3. Composé selon la revendication 1 qui répond à la formule suivante:

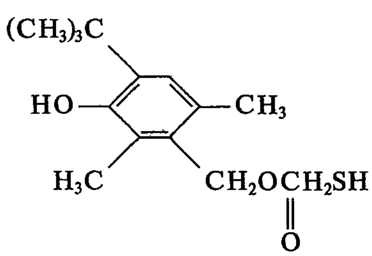

4. Application des composés de formule I selon la revendication 1 comme stabilisants pour matières organiques.

5. Application selon la revendication 4, caractérisé en ce qu'on greffe le composé de formule I sur un polymère en présence d'un générateur de radicaux.

6. Polymères organiques qui ont été stabilisés à l'aide de composés selon la revendication 1.

7. Polymères stabilisés selon la revendication 6, caractérisés en ce qu'ils contiennent des stabilisants à état greffé.